# EUROPEAN PATENT APPLICATION

(11) **EP 1 864 611 A1**
(43) Date of publication of application: **12.12.2007**
(21) Application number: 06730794.2
(22) Date of filing: 31.03.2006
(51) Int. Cl.: A61B 6/03

(54) **BREAST INSPECTION SYSTEM**

(30) Priority: 01.04.2005 JP 2005106933
(71) Applicant: Shibuya, Keizi, Tokushima, 779-0115 (JP)
(72) Inventor: Shibuya, Keizi, Tokushima, 779-0115 (JP)
(74) Representative: HOFFMANN EITLE
(86) International application number: PCT/JP2006/306846
(87) International publication number: WO 2006/106927

(57) **Abstract**

A patient's pain is eliminated and very high diagnostic accuracy is ensured with lower doses of radiation in mammography equipment including: a suction cup (1) having a suction recess (4) disposed, on its interior surface, for being drawn into intimate contact with a chest of the patient (20); a suction member (2) allowing the breast (21) to be drawn from the chest and protruded into the recess (4) in an uncompressed state; and a CT scanner (3) having a storage space (5) to accommodate the suction cup (1) inside and serving to obtain a tomographic image of the breast (21). The breast (21) placed in the storage space (5) via the suction cup (1) is irradiated with the radiation over a plane orthogonal to a protruding direction of the breast (21), and a coronal cross-section image of the breast (21) is obtained in a scanning direction along the protruding direction.

## Description

### TECHNICAL FIELD

The present invention relates to mammography equipment which is used primarily to check for breast cancer. In particular, the present invention pertains to mammography equipment for obtaining a horizontally-sliced, tomographic image of a female breast with very high accuracy while reducing doses of radiation to minimum.

### BACKGROUND ART

The technique of mammography is employed in equipment for detection of breast cancer. In the mammography, a patient's breast is tightly compressed with two parallel plates to reach a thickness of several centimeters, so that the breast is irradiated with x-rays which are, in turn, transmitted through the breast to an x-ray film having been set on one of the compression plates and thus a diagnosis is made in accordance with an image produced on the x-ray film. In the mammography where radiography is carried out after compressing the breast sideway from two directions, it is often difficult to accurately examine a small breast. In order to overcome such difficulty, there has been developed mammography equipment in which a breast is allowed to vertically hang down for compression (refer to Patent Document No. 1). In this particular mammography equipment, however, like in other conventional mammography equipment, the breast is compressed to be thinned and is irradiated with x-rays, which is disadvantageous in that great pains are given to the patient. Especially in order to ensure high examination accuracy in this mammography equipment, the breast has to be forcefully compressed to become flattened. Such forceful compression for flattening the breast causes the patient to suffer even more pains. As such, there exists a trade-off between diagnostic accuracy and patient's pains, and a satisfaction cannot be gained simultaneously in both of the factors.

A patient's pain can be eliminated when a CT scanner is employed. The CT scanner is so designed that the patient is scanned in the direction of her body axis and then a horizontally-sliced image of the breast is reconstructed from a tomographic image of her whole body, so that breast cancer can be screened without the breast being compressed. In the CT scanner, however, since the horizontally-sliced image of the breast is reconstructed through a calculation based on the tomographic image of the patient's whole body, it is difficult to obtain a highly accurate image of the breast. It is because the breast is too small as compared with the whole body in terms of a cross section which is orthogonal to the body axis. The tomographic image of the whole body other than the breast is not essential for examining the breast. Moreover, the too large proportion of the tomographic image of the whole body can lead to decreased diagnostic accuracy for the breast. The CT scanner also carries the disadvantage that doses of radiation become higher as compared with the mammography, and so when the doses of radiation are reduced, the diagnostic accuracy is prone to decrease.

In order to remedy such shortcomings, there has been developed a technique of improving diagnostic accuracy, in which a breast is compressed to narrow a scanning area and correspondingly reduce doses of radiation (refer to Patent Document No. 2).
Patent Document No. 1: Japanese Laid-Open Patent Publication No. 1995-303633
Patent Document No. 2: Japanese Laid-Open Patent Publication No. 1998-328176

### DISCLOSURE OF THE INVENTION

### Problem To Be Solved by the Invention

As shown in Figure 1, according to the Patent Document No. 2, it has a cradle 31 for carrying a patient 20 who is in a lateral recumbent position, the cradle 31 being provided thereon with a pair of compression plates 32 for compressing a breast 21 of the patient 20 sideway from two directions. The compression plate 32 is so arranged as to be transferable in the direction being substantially orthogonal to a plane of the tomographic image, i.e., in the direction of the patient's body axis. With the breast 21 of the patient 20 being compressed with the pair of compression plates 32 in the direction being substantially orthogonal to the plane of the tomographic image, the breast 21 of the patient 20 is diagnosed in the scanning direction along the body axis. This equipment is designed to examine the breast in a compressed state along the scanning direction, allowing a scanning area to be narrowed and an examination to be made with a thinly sliced thickness, so that diagnostic accuracy can be improved with lower doses of radiation.

Nevertheless, this mammography equipment also carries the disadvantage that it is unable to eliminate the influence given to the tomographic image of the breast by the tomographic image of the substantially whole body excluding the breast and that the doses of radiation become higher, relative to the diagnostic accuracy. Also, like in other conventional mammography equipment, the breast has to be compressed to be thinner for improved diagnostic accuracy with lower doses of radiation, which makes it impossible to eliminate the shortcoming of forcing great pains to the patient.

In addition, the image of the breast in a compressed state carries the disadvantage that the breast cancer cannot be detected accurately enough like when the breast is in a natural state. Since breast cancer tends to spread in the horizontal direction which is orthogonal to the protruding direction of the breast, an image of the breast to be examined in a compressed state carries the disadvantage that breast cancer having spread less in the protruding direction and more in the horizontal direction cannot be detected at an earlier stage, which resultantly decreases diagnostic accuracy.

The present invention has, therefore, been made for the purpose of solving such disadvantages. It is the primary object of the present invention to provide ideal mammography equipment for examination of a female breast, in which a patient's pain is mitigated and breast cancer can be detected at an earlier stage, with lower doses of radiation and with very high diagnostic accuracy.

### Means To Solve the Problem

In order to achieve the above-mentioned object, the mammography equipment in accordance with the present invention is constructed and arranged as described below.
The mammography equipment includes a suction cup 1 having a suction recess 4 disposed on its interior surface, the suction recess 4 being drawn into intimate contact with a chest of a patient 20 so as to draw a breast 21 protruding from the chest in an uncompressed state, a suction member 2 drawing the breast 21 into the suction cup 1, and a CT scanner 3 having a storage space 5 for accommodating the suction cup 1 inside. In this mammography equipment, the storage space 5 has the suction cup 1 disposed inside, the suction cup 1 allowing the breast 21 to be placed in the suction recess 4, and the breast 21 is irradiated with radiation by the CT scanner 3 and thus a tomographic image of the breast 21 is obtained. Further, in the mammography equipment, the suction member 2 is operable to draw the breast 21 into the suction recess 4, allowing the breast 21 to be protruded from the chest in an uncompressed state, so that the protruded breast 21 is placed in the storage space 5 via the suction cup 1. This mammography equipment is so constructed and arranged that the CT scanner 3 irradiates the breast 21 with the radiation over a plane which is orthogonal to a protruding direction of the breast 21 and thus a coronal section image of the breast 21 is obtained in a scanning direction along the protruding direction of the breast 21.
To be noted here in the present disclosure is that the coronal section image of the breast is intended to mean the coronal image of the cross-section which graphically slices the breast in the direction orthogonal to the protruding direction. The expression of coronal section image of the breast is used, in a broad sense, to include an oblique coronal image of the cross-section extending obliquely with respect to the plane which is orthogonal to the protruding direction of the breast.

The suction member 2 can be adapted to draw the breast 21 into the suction recess 4 by evacuating air from the suction cup 1. Also, the suction recess 4 in the suction cup can be so interiorly shaped as to draw a single piece of the breast 21, with a recess's horizontal cross-section, which intersects the protruding direction of the breast 21, being either of a circular shape or of an elliptical shape with the ratio of its major diameter to its minor diameter being less than two, and with the horizontal cross-section being tapered off toward a tip of the breast 21.

The suction recess 4 may be provided, on its interior surface, with a contact layer 7 of sol or gel that allows for air-tight contact with a breast surface.

The suction cup 1 may be disposed in a posture where a plane including an edge of an opening of the suction recess 4 is an inclined plane, to allow the breast 21 to be drawn in an oblique direction. The suction cup 1 may also be disposed in a posture where the opening of the suction recess 4 faces downwardly, to allow the breast 21 to be drawn in an upward direction.

The suction recess 4 in the suction cup 1 may be formed with a concavely curved surface that draws at least a portion of the breast 21 into intimate contact with the interior surface of the suction recess 4 and shapes up the breast 21. The suction recess 4 in the suction cup 1 may also be formed with a concavely curved surface that draws a lower surface of the breast 21 into intimate contact with the interior surface of the suction recess 4 and shapes up the breast 21.

The suction cup 1 may be provided with a gasket 6 that tightly contacts a body surface along the edge of the opening of the suction recess 4 so that the edge of the opening of the suction recess 4 tightly contacts a periphery of the breast 21 of the patient 20.

The suction cup 1 may be translucent to allow the breast 21 drawn into the suction recess 4 to be viewed from outside. The suction cup 1 may also be adapted for fabrication with a rubber-like elastic material.

Further, the inventive mammography equipment may be provided with a pair of suction cups 1 that respectively and independently draws both breasts 21 of the patient 20, and the pair of suction cups 1 is linked to the CT scanner 3. Yet further, the suction cup 1 may be formed with an examination hole 11 through which a biopsy needle 12 is inserted.

### Effect of the Invention

The mammography equipment in accordance with a first aspect of the present invention has the advantage that while the patient's pain is eliminated, the breast cancer can be detected at an earlier stage with lower doses of radiation and with very high diagnostic accuracy. It is because the inventive mammography equipment is so constructed and arranged that the suction cup is drawn into intimate contact with the patient's chest, the air is evacuated from the suction cup by the suction member, the breast is drawn into the suction recess so as to be protruded from the chest in an uncompressed state, as well as the protruded breast is placed in the storage space of the CT scanner to obtain the tomographic image of the breast by means of the CT scanner. Unlike in other conventional equipment, the mammography equipment thus structured is able to carry out the examination, eliminating the patient's pain, because the tomographic image of the breast is obtained without compressing the breast to be thinned by the compression plates. In addition, the inventive equipment is designed to image the breast by drawing the breast into the suction recess and shaping up the breast to a natural form, without compression, so that the shape and size of cancer and its location can be accurately detected. Further, since the inventive mammography equipment is designed to draw the breast into the suction recess in the suction cup and allow the breast to be protruded from the chest, even a smaller breast can be sufficiently protruded from the chest to obtain a well-accepted image.

Further, the inventive mammography equipment is so constructed and arranged that the CT scanner irradiates the breast with the radiation over the plane which is orthogonal to the protruding direction of the breast and thus the coronal section image of the breast is obtained in the scanning direction along the protruding direction of the breast, so that the tomographic image of the breast can be obtained by irradiating the patient's breast portion with the radiation while the doses of radiation are reduced. And, a portion other than the breast is not irradiated with the radiation, so that the tomographic image of the breast is not to be affected by a tomographic image of the portion other than the breast. Further, this mammography equipment is designed to scan the breast in the scanning direction along the protruding direction of the breast, so that an ideal image can be obtained of a breast cancer that has spread in the direction where breast cancer is very likely to spread, namely, in the direction which is orthogonal to the protruding direction of the breast, and thus high diagnostic accuracy can be ensured.

The mammography equipment in accordance with a second aspect of the present invention is advantageous in that the suction recess is so interiorly shaped as to draw the single piece of the breast, with the recess's horizontal cross-section, which intersects the protruding direction of the breast, being either of a circular shape or of an elliptical shape, and with the horizontal cross-section being tapered off toward the tip of the breast, so that the breast drawn into the suction recess can be shaped up to a more natural form.

The mammography equipment in accordance with a third aspect of the present invention is advantageous in that the suction recess is provided, on its interior surface, with a contact layer of sol or gel, allowing the breast to be securely drawn into intimate contact with the interior surface of the suction recess and to be shaped up to a neater form.

The mammography equipment in accordance with a fourth aspect of the present invention is advantageous in that the suction cup is disposed in the posture where the plane including the edge of the opening of the suction recess is the inclined plane, allowing the breast to be drawn in the oblique direction, so that the breast hanging down under the effect of gravity can be drawn into the suction recess and protruded sufficiently from the patient's chest to carry out the examination. In particular, the mammography equipment is also advantageous in that it can be designed to examine the breast, with the patient being in a forwardly leaning posture, so that a cost of manufacture can be reduced with an overall, compact configuration of the equipment without requiring extra facilities such as a bed.

The mammography equipment in accordance with a fifth aspect of the present invention is advantageous in that the suction cup is disposed in the posture where the opening of the suction recess faces downwardly, being structured to allow the breast to be drawn in an upward direction, so that the patient can be examined in a comfortable, supine posture on a bed or the like.

The mammography equipment in accordance with sixth and seventh aspects of the present invention is advantageous in that the suction recess in the suction cup is formed with a concavely curved surface that draws at least a portion of or the lower surface of the breast into intimate contact with its interior surface and shapes up the breast, so that the breast can be shaped up to a more ideal form for examination.

The mammography equipment in accordance with an eighth aspect of the present invention is provided with the gasket along the edge of the opening of the suction recess in the suction cup, allowing the edge of the opening of the suction recess to tightly contact the body surface around the patient's breast, so that the air can be efficiently evacuated from the suction cup to draw the breast into the suction cup.

The mammography equipment in accordance with a ninth aspect of the present invention is advantageous in that the suction cup is translucent to allow the breast drawn into the suction recess to be viewed from outside, so that the breast can be shaped up to a natural form for examination, facilitating monitoring the shape of the breast to be drawn into the suction recess.

Further, the mammography equipment in accordance with a tenth aspect of the present invention is provided with a pair of suction cups that respectively and independently draws both breasts of the patient, and the pair of suction cups is linked to the CT scanner, so that both of the breasts can be simultaneously examined by irradiating the two breasts with the radiation for transmission through the breasts. The mammography equipment is, therefore, advantageous in that an examination of two breasts can be expedited within a shorter period of time.

### BEST MODE FOR CARRYING OUT THE INVENTION

Embodiments of the present invention will now be discussed in conjunction with the accompanying drawings. It should be noted here that the following embodiments are intended to be illustrative of mammography equipment to embody the technical ideas of the invention, and that the inventive mammography equipment is, in no way, limited to what is described below.

Further, in order to facilitate understanding the Claims in the present disclosure, the reference numerals corresponding to the members shown in the Embodiments are appended to the members shown both in the Claims and in the Means To Solve the Problem. It should also be noted that the members shown in the Claims are, in no way, limited to the members shown in the embodiments.

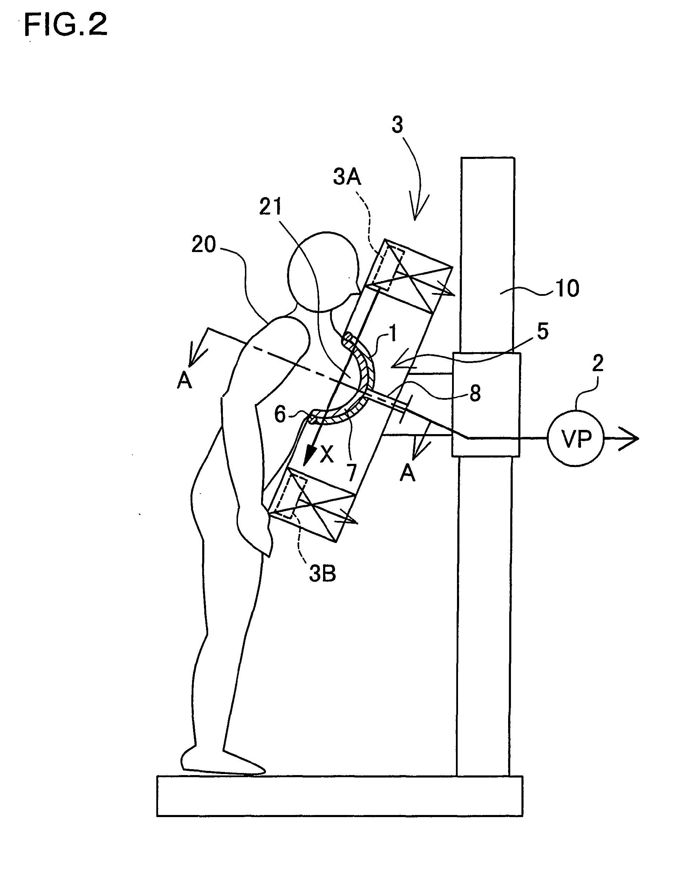

The mammography equipment shown in Figures 2 through 7 includes a suction cup 1 having a suction recess 4 disposed on its interior surface, the suction recess 4 being drawn into intimate contact with a chest of a patient 20 so as to draw a breast 21 protruding from the chest in an uncompressed state, a suction member 2 drawing the breast 21 into the suction recess 4 by evacuating air from the suction cup 1, and a CT scanner 3 having a storage space 5 for accommodating the suction cup 1 inside.

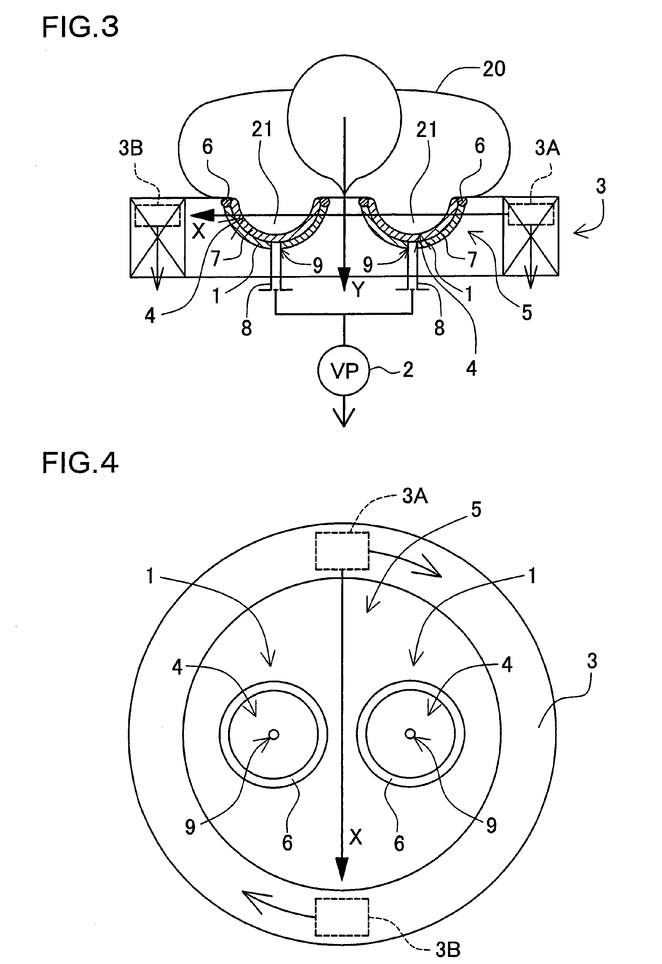

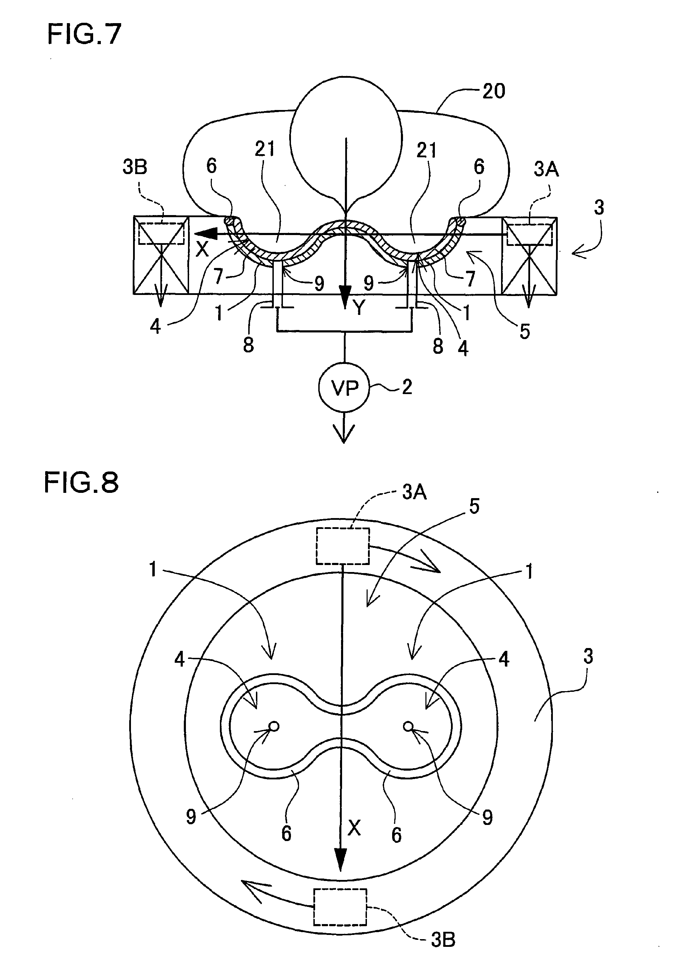

The suction cup 1 shown in Figures 2 through 6 is designed to draw a single piece of the breast 21 into the suction recess 4, while the suction cup 1 in Figures 7 and 8 is designed to draw both breasts 21 into the suction recess 4. In the suction recess 4 drawing the breast 21, the horizontal cross-section, which intersects the protruding direction of the breast 21 (the direction which is orthogonal to the body axis, substantially a front direction of the patient 20), is of a circular shape as shown in Figure 4. It should be noted that, although not shown, the horizontal cross section of the suction recess may also be of an elliptical shape with the ratio of its major diameter to its minor diameter being less than two, which is closer to a circular shape.

Further, the suction recess 4 is so designed that the horizontal cross-section shape is tapered off toward the tip of the breast 21 so that the breast 21 is drawn and shaped up to a natural form. The illustrated suction cup 1 is so designed that the shape of the suction recess 4 corresponds to a hemisphere or cone. The suction cup 1 with this shape is able to draw the breast surface into the suction recess 4 in a wide area, for example, more than 50% of the breast surface and shape up the breast to a natural form. Especially in the case of a suction cup which allows more than 80% of the breast surface to be drawn into the suction recess, the breast can be shaped up to an ideal form. Thus, the equipment in which the breast 21 is drawn to the interior surface of the suction cup 1 and examined in a shaped-up state is able to keep the shape of the breast 21 stabilized for examination, so that the examination accuracy can be advantageously increased. It is because examination accuracy decreases when a breast moves during a short period of a few seconds in examination.

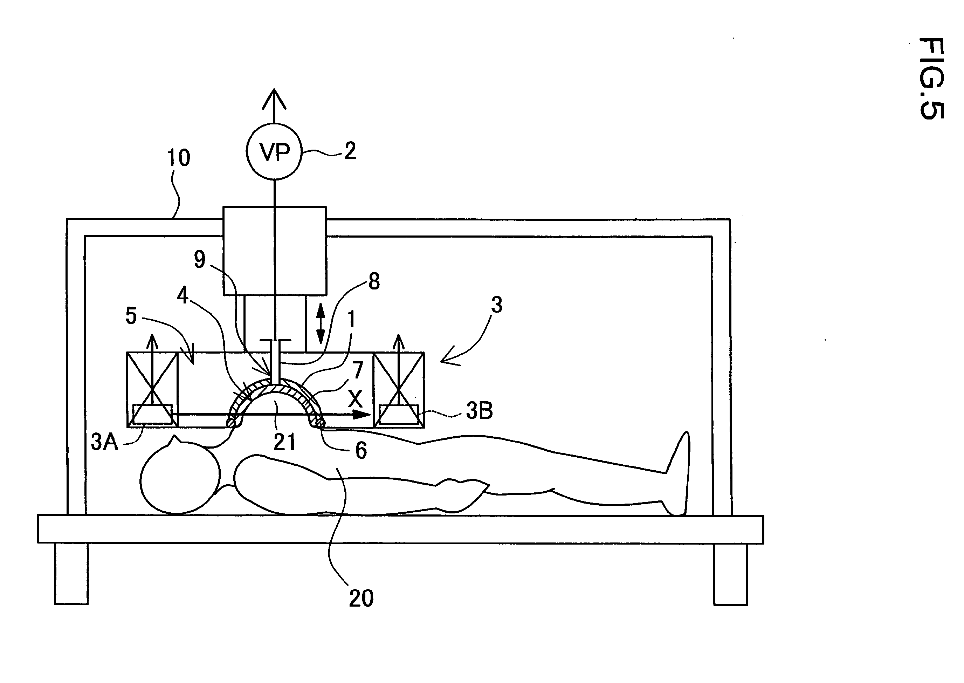

When used for a patient 20 in a forwardly leaning posture, as shown in Figure 2, the mammography equipment drawing a portion of the breast 21 to the interior surface of the suction recess 4 allows the lower surface of the breast 21 to be drawn to the interior surface of the suction recess 4. The suction cup 1 draws the lower surface of the breast 21 into intimate contact with the suction recess 4 and shapes up the breast, so that the breast 21 substantially in its entirety can be shaped up to a natural form. When used for the patient 20 in a supine position, as shown in Figure 5, the mammography equipment allows the periphery of the breast 21 to be drawn into intimate contact with the interior surface of the suction recess 4 and allows the substantially entire breast 21 to be shaped up to a natural form.

Preferably, however, the suction cup 1 is so shaped that more than 50%, preferably more than 80%, of the breast surface is drawn into intimate contact with the interior surface of the suction recess 4 and shaped up. It is because the intimate contact can be obtained when the entire breast is shaped up to a more natural and ideal form. The suction cup 1 drawing the breast surface into intimate contact with the suction recess 4 in a wide area is arranged to be interchangeable in accordance with a size of the breast 21 of the patient 20, because the size of the breast 21 of the patient 20 varies from individual to individual. A suction cup with a larger suction recess is used for a patient with a larger breast, while a suction cup with a smaller suction recess is used for a patient with a smaller breast.

The illustrated suction cup 1 is provided, along the edge of the opening of the suction recess 4, with the gasket 6 that tightly contacts the body surface so that the edge of the opening of the suction recess 4 may tightly contact the periphery of the breast 21 of the patient 20. The gasket 6 is of a rubber-like elastic material deforming elastically or of a gelated material having an adherence. In the suction cup 1 shown in Figures 3, 4 and 6, the gasket 6 is of a substantially circular shape. The suction cup 1 uses the gasket 6 to allow the edge of the opening of the respective suction recess 4 to tightly contact the periphery of the respective breast 21.

The gasket 6 shown in Figures 7 and 8 is shaped like a gourd having two circular arcs connected laterally with each other. The gasket 6 is so designed that both breasts 21 are placed inside, in other words, the edge of the opening of the suction recess 4 is allowed to tightly contact the peripheries of the two breasts 21, so that the two breasts 21 are drawn into the suction recess 4 and shaped up. The suction cup 1 draws the two breasts 21 into the suction recess 4 and shapes up the respective breast 21 to a natural form. Thus, the suction recess 4 in the suction cup 1 is designed to draw more than 50%, preferably more than 80%, of the respective breast surface into intimate contact with the interior surface and shape up each breast to a natural form.

The illustrated suction cup 1 is so designed that the suction recess 4 is provided, on its interior surface, with a contact layer 7 of sol or gel to allow for air-tight contact with the breast surface. The suction cup 1 has the feature that the breast 21 is securely drawn into intimate contact with the interior surface of the suction recess 4 and shaped up to a neater form. The contact layer 7 allows the interior surface of the suction recess 4 to contact the breast surface in an air-tight manner. As such, when the air is evacuated from the suction recess 4, the breast 21 can contact the interior surface of the suction recess 4 in a more secure manner. It should be noted, however, that the suction cup does not necessarily have to be provided, on its interior surface, with such a contact layer. It is because the edge of the opening of the suction recess 4 can contact the body surface of the patient 20 and the breast 21 can be in intimate contact with the suction recess 4.

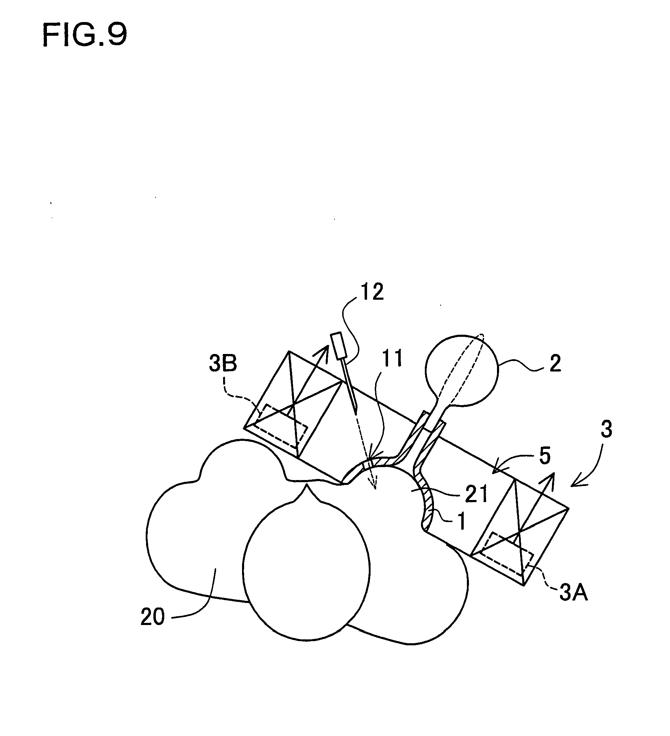

The suction cup 1 shown in Figure 9 is connected to the suction member 2 that is of a hollow body made of a rubber-like elastic material being elastically deformable. The illustrated suction member 2 is of a hollow sphere made of the rubber-like elastic material. When the spherical suction member 2 is compressed, that is to say, when the air is evacuated from the suction member, the suction cup 1 is caused to tightly contact the surface of breast 21 Subsequently, with the breast 21 being allowed to tightly contact the suction cup 1, when the suction member 2 is released from its compressed state, the air is evacuated from the suction cup 1 by the effect of the restoring force of the rubber-like elastic material, and the breast 21 is drawn to be protruded. The suction cup 1 is able to easily draw the breast without having to be connected to a suction member such as a vacuum pump. It should be noted, however, that this suction cup is also able to draw the breast inside when connected to a suction member of a suction pump to evacuate the air from inside.

The suction cup 1 is translucent to allow the shape of the breast 21 drawn into the suction recess 4 to be viewed from outside. The suction cup 1 is made of radiotransparent glass. In place of glass, however, the suction cup 1 may also be made of a radiotransparent plastic material. The suction cup 1 has the feature that it allows the shape of the breast 21 being drawn to the suction recess 4 to be monitored, so that the breast 21 can be shaped up to a natural state for examination.

Further, the suction cup 1, as shown in Figure 9, is formed with an examination hole 11 through which a biopsy needle 12 is inserted. The examination hole 11 is extended through the suction cup 1 but is bored to such a size as may be hermetically closed by the breast 21 when the breast 21 is drawn to the interior surface. Therefore, while the breast 21 is drawn into the suction cup 1, the open air does not infiltrate through the examination hole 11 into the suction cup 1. The suction cup 1 is so designed that while the breast 21 is drawn and protruded, the biopsy needle 12 is inserted through the examination hole 11, so that a cell sample can be collected from a specific site within the breast 21. The breast subjected to an examination by using the mammography equipment is sometimes examined by collecting a cell sample from a specific site. In such a case, the breast 21 is first examined while being drawn to the suction cup 1, and then the breast 21 is kept in the state of having been drawn to the suction cup 1, and further the biopsy needle12 is inserted through the examination hole 11, so that the cell sample can be collected from a precise site in the breast 21. The suction cup 1 shown in Figure 9 is, in its entirety, made of a rubber-like elastic material, but the examination hole can be formed in any suction cup designed for drawing the breast inside. In this case, the examination hole is bored to such a size as may be closed by the breast being drawn to the interior surface so that the open air may not be drawn in. The examination holes may be bored in a plurality of spots so that the cell sample can be precisely collected from a specific site in the breast.

In the mammography equipment shown in Figure 2, the breast 21 is examined when the patient 20 is in her forwardly leaning posture. The mammography equipment is so designed that the suction cup 1 is disposed in the posture where the plane including the edge of the opening of the suction recess 4 is the inclined plane, allowing the breast 21 to be drawn in the downwardly oblique direction. The mammography equipment is so designed that the lower surface of the breast 21 is drawn into intimate contact with the suction recess 4 and the breast is shaped up. The mammography equipment is also so designed that the breast 21 hanging down under the effect of gravity is drawn into the suction recess 4 and shaped up. As such, an examination can be carried out by allowing the breast 21 to be sufficiently protruded from the body surface of the patient 20.

The mammography equipment shown in Figure 5 is so designed that the breast 21 is examined when the patient 20 is in her supine posture. In this mammography equipment, the suction cup 1 is disposed in the posture where the opening of the suction recess 4 faces downwardly, and so the breast 21 is examined by being drawn upwardly into the suction recess 4 in the suction cup 1. The mammography equipment allows the breast 21 to be examined while the patient 20 is placed in a comfortable posture. Also, since the breast 21 is examined by being drawn upwardly, an operator's workability can be improved as well.

The suction cup 1 is linked via a linkage mechanism (not shown) to the CT scanner 3, or alternatively to the frame 10, to be placed in a specific posture. However, the suction cup 1 does not necessarily have to be linked to the CT scanner 3 or the frame 10. It is because the breast 21 can be examined by adjusting the relative position between the patient 20 and the CT scanner 3 so that the suction cup 1 drawing the breast 21 is positioned in the storage space 5 in the CT scanner 3.

The suction member 2 evacuates the air from the suction cup 1 and draws the breast 21 into the suction recess 4. A suction pump such as a vacuum pump can be used with the suction member 2. The suction member 2 is linked via a suction hose 8 to the suction recess 4 in the suction cup 1. The suction recess 4 is formed with a through hole 9 at its tip, and the suction hose 8 is linked to the through hole 9. The suction member 2 evacuates the air from the suction cup 1 to such a degree of vacuum as may draw the breast 21 into the suction recess 4.

The CT scanner 3 is provided with the storage space 5 for accommodating the suction cup 1 inside. The illustrated CT scanner 3 is arranged to be annular in its entirety and is provided with the storage space 5 inside. Although not shown, the CT scanner may also be formed with a recess so that the recess may serve as a storage space for the suction cup. The suction cup 1 drawing the breast 21 is placed in the storage space 5, and as indicated at arrow X in the drawings, the breast 21 is irradiated with the radiation such as x-rays and gamma rays from the periphery of the breast 21 to horizontally extend through the breast 21, so that the coronal section image of the breast 21 is obtained.

The CT scanner 3, in its annular casing, includes an x-ray irradiator 3A irradiating the breast with radiation such as x-rays and gamma rays, an x-ray detector 3B receiving and detecting the x-ray radiation emitted from the x-ray irradiator 3A for x-ray transmission through the breast, a calculator (not shown) calculating the coronal section image of the breast based on the density of the x-rays which are detected by the x-ray detector 3B, and a driving mechanism (not shown) having the x-ray irradiator 3A and x-ray detector 3B placed in their opposite positions and allowing for a scanning procedure in a rotation around the breast and in a shift in the direction of Y axis. The CT scanner 3 obtains the coronal section image of the breast by rotating the x-ray irradiator 3A and x-ray detector 3B within the casing while maintaining the x-ray irradiator 3A and x-ray detector 3B in their opposite positions. The CT scanner 3 obtains a plurality of coronal section images of the breast 21 which has graphically undergone the horizontal slicing procedure from the tip to the body surface in a scanning direction along the protruding direction of the breast 21 as indicated at arrow Y in the drawings. In the CT scanner 3, the radiation is not emitted so as to be transmitted throughout the whole body. The breast 21 is irradiated with the radiation for horizontal transmission. That is to say, the breast 21 is focally irradiated with the radiation.

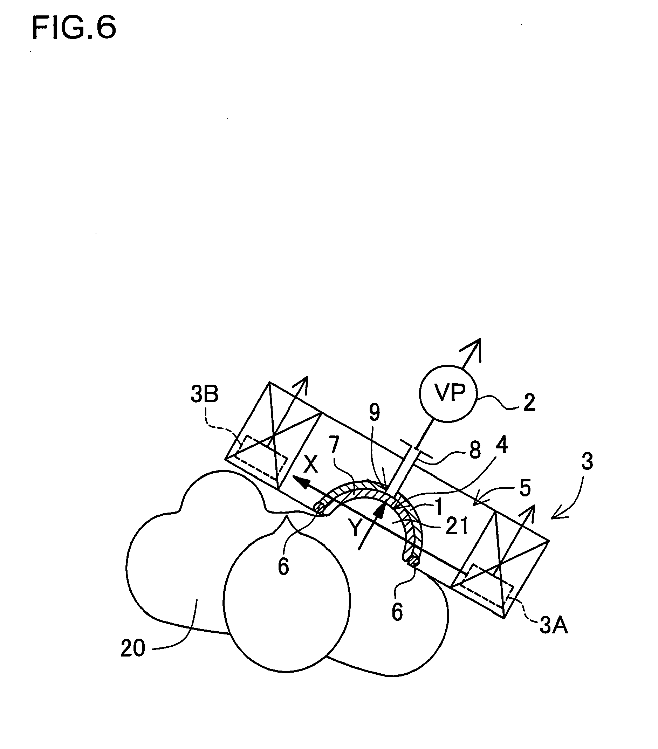

The CT scanner 3 shown in Figures 2 through 5 has a pair of suction cups 1 disposed in the storage space 5. Therefore, the CT scanner 3 examines both breasts 21 simultaneously by irradiating the two breasts 21 with the radiation for transmission through the two breasts. Since the two breasts 21 are simultaneously examined in this mammography equipment, the examination of two breasts 21 can be expedited within a shorter period of time. The CT scanner 3 shown in Figure 6, on the other hand, has a single suction cup 1 disposed in the storage space 5, and the radiation is emitted for transmission through a single piece of the breast 21. This mammography equipment is able to examine the breast 21 in higher diagnostic accuracy by examining each individual breast 21 independently. Especially, because the single piece of the breast 21 is drawn into the suction cup 1 to be disposed in the storage space 5 in the CT scanner 3, the entirety of the breast 21, in particular a portion near the sternum as well, is to be disposed in the storage space 3, so that the mammography equipment has the feature that the entire breast can be examined in an ideal state. Also, since the radiation is emitted for transmission through the single piece of the breast 21, the breast 21 can be examined in high diagnostic accuracy with lower doses of radiation.

The CT scanner 3 is linked via the linkage mechanism to the frame 10 so as to be disposed in a specific posture and location with respect to the patient 20. The linkage mechanism allows the CT scanner 3 to be linked to the frame 10 so that the location and posture of the CT scanner 3 may be adjustable.

The above-described mammography equipment is designed to examine the breast 21 of the patient 20 in the following procedure.
(1) The breast 21 of the patient 20 is covered by the suction cup 1, as shown in Figure 2 or Figure 5.
(2) The suction member 2 evacuates the air from within the suction cup 1 to draw the breast 21 into the suction recess 4 in the suction cup 1. The drawn breast 21 is drawn into intimate contact with the interior surface of the suction cup 1 and shaped up by the suction cup 1.
(3) The suction cup 1 is placed in the storage space 5 in the CT scanner 3. At this moment, the relative position between the CT scanner 3 and the patient 20 is adjusted, and the suction cup 1 is placed in the storage space 5 in the CT scanner 3.
(4) The CT scanner 3 irradiates the breast 21, drawn into the suction cup 1, with the radiation. The CT scanner 3 irradiates the breast 21 with the radiation from the periphery of the breast 21, as indicated at arrow X in the drawings, and further scans the breast 21 along the protruding direction of the breast 21, as indicated at arrow Y, so that the coronal section image of the entire breast is calculated and displayed on a monitor (not shown). That is to say, a plurality of coronal section images are produced, based on horizontal graphical slices of he breast 21, and the entire breast is examined based on each coronal section image. A mammary cancer within the breast 21 is displayed in the coronal section image which contains the breast cancer.

### INDUSTRIAL APPLICABILITY

The present invention enables breast cancer to be detected with lower doses of radiation and with very high accuracy while eliminating a patient's pain, so that a large number of women are relieved to have a safe detection of breast cancer. Thus, a detection of breast cancer can be promoted among a great number of women to ensure earlier detection of breast cancer and decrease a death rate associated with the breast cancer.

### BRIEF DESCRIPTION OF THE DRAWINGS

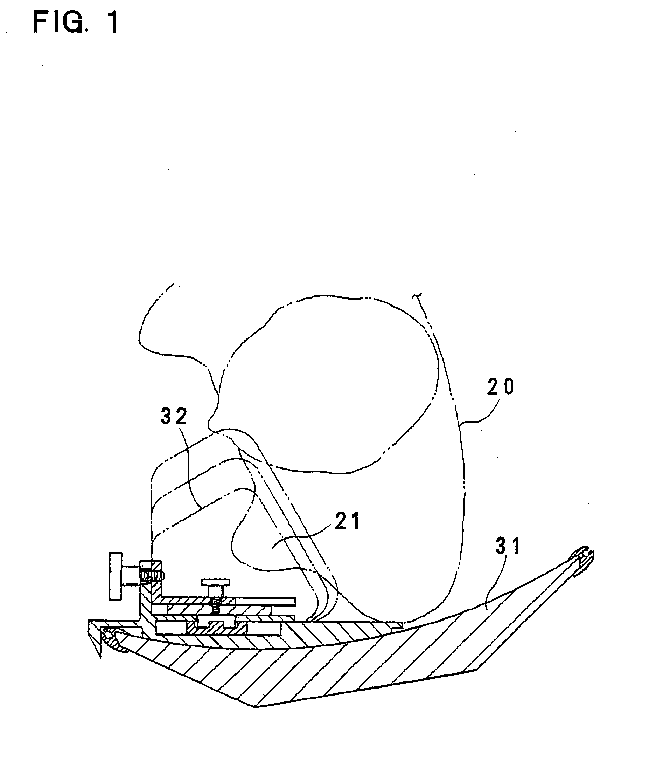

Figure 1 is a cross-sectional view showing conventional mammography equipment in operative position;
Figure 2 is a cross-sectional view showing the mammography equipment in operative position in accordance with an embodiment of the present invention;
Figure 3 is a cross-sectional view, taken along lines A--A, showing the mammography equipment illustrated in Figure 2;
Figure 4 is a front view, as viewed from the patient's side, showing the CT scanner and suction cup in the mammography equipment illustrated in Figure 2;
Figure 5 is a cross-sectional view showing the mammography equipment in operative position in accordance with another embodiment of the present invention;
Figure 6 is a cross-sectional view showing the mammography equipment in operative position in accordance with a further embodiment of the present invention;
Figure 7 is a cross-sectional view showing the mammography equipment in operative position in accordance with an even further embodiment of the present invention;
Figure 8 is a front view, as viewed from the patient's side, showing the CT scanner and suction cup in the mammography equipment illustrated in Figure 7; and
Figure 9 is a cross-section view showing the mammography equipment in operative position in accordance with an alternative embodiment of the present invention.

### DENOTATION OF REFERENCE NUMERALS

- 1: Suction Cup
- 2: Suction Member
- 3: CT Scanner
- 3A: X-ray Irradiator
- 3B: X-ray Detector
- 4: Suction Recess
- 5: Storage Space
- 6: Gasket
- 7: Contact Layer
- 8: Suction Hose
- 9: Through Hole
- 10: Frame
- 11: Examination Hole
- 12: Biopsy Needle
- 20: Patient
- 21: Breast
- 31: Cradle
- 32: Compression Plate

## Claims

1. Mammography equipment comprising:
a suction cup (1) having a suction recess (4) disposed on an interior surface thereof, the suction recess (4) being operable to be drawn into intimate contact with a chest of a patient (20) so as to draw her breast (21) protruding from the chest in an uncompressed state;
a suction member (2) drawing the breast (21) into the suction cup (1); and
a CT scanner (3) having a storage space (5) for accommodating the suction cup (1) inside,
wherein the suction cup (1) is disposed inside the storage space (5), the suction cup (1) allowing the breast (21) to be placed in the suction recess (4), and the breast (21) is irradiated with radiation by the CT scanner (3) in order to obtain a tomographic image of the breast (21),
wherein the suction member (2) is operable to draw the breast (21) into the suction recess (4), allowing the breast (21) to be protruded from the chest in an uncompressed state, so that the protruded breast (21) is placed in the storage space (5) via the suction cup (1), and
wherein the CT scanner (3) irradiates the breast (21) with the radiation over a plane being orthogonal to a protruding direction of the breast (21) and thus a coronal section image of the breast (21) is obtained in a scanning direction along the protruding direction of the breast (21).

2. The mammography equipment as recited in claim 1 wherein the suction member (2) is adapted to draw the breast (21) into the suction recess (4) by evacuating air from the suction cup (1).

3. The mammography equipment as recited in claim 1 wherein the suction recess (4) is so interiorly shaped as to draw a single piece of the breast (21), with a recess's horizontal cross-section, which intersects the protruding direction of the breast (21), being either of a circular shape or of an elliptical shape with the ratio of the major diameter to the minor diameter being less than two, and with the horizontal cross-section being tapered off toward a tip of the breast (21).

4. The mammography equipment as recited in claim 1 wherein the suction recess (4) is provided, on an interior surface thereof, with a contact layer (7) of sol or gel to allow for air-tight contact with a breast surface.

5. The mammography equipment as recited in claim 1 wherein the suction cup (1) is disposed in a posture where a plane including an edge of an opening of the suction recess (4) is an inclined plane, to allow the breast (21) to be drawn in an oblique direction.

6. The mammography equipment as recited in claim 1 wherein the suction cup (1) is disposed in a posture where the opening of the suction recess (4) faces downwardly to allow the breast (21) to be drawn in an upward direction.

7. The mammography equipment as recited in claim 1 wherein the suction recess (4) in the suction cup (1) is formed with a concavely curved surface that draws at least a portion of the breast (21) into intimate contact with the interior surface of the suction recess (4) and shapes up the breast (21).

8. The mammography equipment as recited in claim 1 wherein the suction recess (4) in the suction cup (1) is formed with a concavely curved surface that draws a lower surface of the breast (21) into intimate contact with the interior surface of the suction recess (4) and shapes up the breast (21).

9. The mammography equipment as recited in claim 1 wherein the suction cup (1) is provided with a gasket (6) that tightly contacts a body surface along the edge of the opening of the suction recess (4) so that the edge of the opening of the suction recess (4) tightly contacts a periphery of the breast (21) of the patient (20).

10. The mammography equipment as recited in claim 1 wherein the suction cup (1) is translucent to allow the breast (21) drawn into the suction recess (4) to be viewed from outside.

11. The mammography equipment as recited in claim 1 wherein the suction cup (1) is of a rubber-like elastic material.

12. The mammography equipment as recited in claim 1 further comprising a pair of suction cups (1) that respectively and independently draws both breasts (21) of the patient (20), and wherein the pair of suction cups (1) is linked to the CT scanner (3).

13. The mammography equipment as recited in claim 1 wherein the suction cup (1) is formed with an examination hole (11) through which a biopsy needle (12) is inserted.
